# EUROPEAN PATENT APPLICATION

(11) **EP 4 086 352 A1**
(43) Date of publication of application: **09.11.2022**
(21) Application number: 21172894.4
(22) Date of filing: 07.05.2021
(51) Int. Cl.: C12N 15/867, C07K 14/15

(54) **GLYCOPROTEINS FOR PSEUDOTYPING RETROVIRAL VECTOR PARTICLES**

(71) Applicant: Medizinische Hochschule Hannover, 30625 Hannover (DE)
(72) Inventor: Schambach, Axel, 30625 Hannover (DE); Schott, Juliane, 30625 Hannover (DE); Wolf, Susanne, 30419 Hannover (DE)
(74) Representative: Taruttis, Stefan Georg

(57) **Abstract**

The invention provides retroviral envelope glycoproteins in which the cytoplasmic C-terminal tail (CTT) from N-terminus to C-terminus comprises or consists of a T-domain and an R-domain, wherein the R-domain is truncated from its C-terminus, and hybrid glycoproteins and retroviral, especially alpharetroviral, lentiviral or gammaretroviral vector particles containing the glycoprotein. The glycoproteins target the SLC1A5 receptor for target cell entry.

## Description

The present invention provides pseudotyped retroviral vector particles, especially alpharetroviral vector particles or lentiviral vector particles, having high tropism for human cells, e.g. human blood cells, and having high transduction efficiency for human cells, a process for producing the pseudotyped retroviral vector particles with a high titer from packaging cells, as well as the glycoproteins that are used for pseudotyping. To pseudotype the retroviral vector particles for altering their tropism or increasing their specificity for human cells, the glycoprotein of the invention is incorporated into the envelope lipid bilayer of the retroviral vector particle during its production in packaging cells.

The invention provides glycoproteins suitable for altering the tropism or improving the delivery or entry of retroviral, especially alpharetroviral and lentiviral, particles using novel glycoproteins or glycoproteins that are artificial hybrids of different viral origin, and which preferably allow for particle production at increased titers when generating the retroviral vector particles in packaging cells, especially in human packaging cells. Further, the retroviral envelope glycoproteins provide the vector particles with a high tropism for human cells, and a high efficiency for use in transducing human cells, especially NK cells, T-cells and hematopoietic stem cells

### State of the art

Graham et al., J. gen. Virol. 36, 59-72 (1977) and Pear et al., PNAS 8392-8396 (1993) describe the packaging cell line termed HEK293T-cells suitable for producing retroviral vector particles.

EP 1 499 736 B1 claims a chimeric glycoprotein, which is also referred to as RD114/TR, that comprises a cytoplasmic tail domain from MLV-A and a transmembrane and extracellular domain from feline endogenous retrovirus RD114, and describes the glycoprotein as suitable for pseudotyping lentiviral vector particles for tropism towards human hematopoietic stem cells.

It is generally known that different retroviruses in their outer envelope have integrated glycoproteins that have large differences or are structurally unrelated.

### Object of the invention

It is an object of the invention to provide alternative glycoproteins suitable for pseudotyping retroviral vector particles, which glycoproteins shall be suitable for gene therapy, e.g. due to a high tropism for human cells, provide a high transduction efficiency of human cells, and which glycoproteins allow for the production of the retroviral vector particles at a high titer in packaging cells.

### Description of the invention

The invention achieves the object by the features of the claims, and especially by providing retroviral envelope glycoproteins in which the cytoplasmic C-terminal tail (CTT) from N-terminus to C-terminus comprises or consists of a T-domain and an R-domain, wherein the R-domain is truncated from its C-terminus, and retroviral, especially alpharetroviral, lentiviral or gammaretroviral vector particles containing the glycoprotein. In an alternative, the motif forming the protease site, at which T and R are separated upon particle maturation, is replaced by a heterologous or an artificial sequence motif. The CTT has a protease recognition motif that overlaps the C-terminal region of the T-domain and the adjacent N-terminal region of the R-domain. During maturation of the viral particle during/after budding and prior to entry of the viral vector into a cell, the R-domain is cleaved off the T-domain at the protease recognition motif, and this cleavage is needed for efficient cell entry. In a further alternative, the glycoproteins are hybrids with the ectodomain and the membrane-spanning domain derived from a different retrovirus than the CTT.

Surprisingly, the glycoproteins of the invention were integrated into the retroviral vector particles and provide for tropism towards human cells, especially towards NK cells, T-cells and hematopoietic stem cells, and the vector particles pseudotyped with the glycoproteins having a C-terminally truncated CTT are produced in packaging cells to a high titer. Currently, the tropism of the glycoproteins of the invention for human cells is believed to be based on the glycoproteins binding to human cell-surface receptors that are expressed on several cells. One of the receptors that are assumed to be bound by the glycoproteins is SLC1A5 in the case of glycoproteins containing one of the ectodomains of RD114, BaEV and MPMV. All of the glycoproteins described herein, including its truncation variants and hybrid variants, as a common feature target the SLC1A5 receptor for target cell entry. The viral vector particles of the invention are suitable for use in the genetic treatment of human NK cells, T-cells and hematopoietic stem cells that have a genetic defect, especially for use in the treatment of the following diseases: Globinopathies, metabolic diseases, infectious diseases, immunodeficiencies. Further, the viral vectors of the invention can be used for treatment by genetic manipulation of tissues and cells, e.g. treatment of liver, neurons, inner ear, retina, or skin.

Generally herein, arrangements of glycoprotein domains are given from N-terminus to C-terminus. Glycosylation of the proteins occurs during expression from nucleic acid constructs encoding for the glycoprotein, e.g. during expression in packaging cells.

The retroviral envelope glycoprotein that contains the CTT from N-terminus to C-terminus is comprised of a surface unit or ectodomain, a transmembrane domain, and a CTT which is composed of the T-domain and the R-domain, which originates from RD114 and is C-terminally truncated, or the R-domain originates from BaEV and can be C-terminally truncated, or which CTT originates from MPMV or MLV-A, or which consists of only the T domain of MLV-A, i.e. without any R domain.

The glycoproteins have been found to be suitable for pseudotyping alpharetroviral vector particles, e.g. based on ASLV, or lentiviral vector particles or gammaretroviral vector particles.

In a first embodiment, the CTT originates from RD114 and it is truncated from its C-terminus, especially truncated for 1 C-terminal amino acid, leaving 16 N-terminal amino acids of the R-domain, or truncated for its 8 or for 9 C-terminal amino acids, leaving 9, respectively 8 N-terminal amino acids of the R-domain, wherein the retroviral envelope glycoproteins preferably are present in alpharetroviral vector particles. In this embodiment, the C-terminal portion of the natural CTT is truncated. As the R-domain is the C-terminal portion of the CTT, the R-domain is truncated. In the embodiment, the CTT, respectively the R-domain, can be truncated for 9 C-terminal amino acids, leaving 8 N-terminal amino acids of the R-domain, or truncated for its 14 or for 15 C-terminal amino acids, leaving 3, respectively 2 N-terminal amino acids of the CTT, wherein the retroviral envelope glycoproteins preferably are present in lentiviral vector particles. In the embodiment, the CTT can consist of the T-domain of RD114, corresponding to a complete deletion of the R-domain. In the first embodiment, the ectodomain and membrane spanning domain preferably are from RD114.

In the first embodiment, the glycoprotein from N-terminus to C-terminus consists of an ectodomain of SEQ ID NO: 1, a transmembrane domain of SEQ ID NO: 2, and a CTT of one of SEQ ID NO: 3, SEQ ID NO: 4, SEQ ID NO: 11, SEQ ID NO: 12, SEQ ID NO: 17, SEQ ID NO: 18, and SEQ ID NO: 20, wherein for alpharetroviral vector particles a CTT of one of SEQ ID NO: 4, SEQ ID NO: 11, SEQ ID NO: 12, and SEQ ID NO: 20 is preferred, and for lentiviral vector particles a CTT of one of SEQ ID NO: 17, SEQ ID NO: 18, and SEQ ID NO: 12 is preferred.

Optionally, within the CTT, overlapping the C-terminus of its T-domain and the N-terminus of the R-domain, the protease motif can be replaced by an artificial or heterologous protease motif, e.g. a protease motif from ASLV replacing the same number of C-terminal amino acids of the T-domain and of N-terminal amino acids of the R-domain as in SEQ ID NO: 41, with the remaining R-domain being joined to the protease motif, or a synthetic protease motif as in SEQ ID NO: 42.

In a second embodiment, the glycoproteins are derivatives of the envelope glycoprotein of Baboon endogenous retrovirus (BaEV), wherein the C-terminal portion of the natural CTT is truncated. This glycoprotein from N-terminus to C-terminus consists of an ectodomain of SEQ ID NO: 21, a transmembrane domain of SEQ ID NO: 22, and a C-terminally truncated CTT. This BaEV-derived glycoprotein has a C-terminal truncation of its C-terminal 9 to 17 amino acids, which truncation is in the C-terminal CTT, preferably the truncation extending to the 11 to 16 C-terminal amino acids of the CTT. The truncated CTT is one of SEQ ID NO: 34 having a C-terminal truncation of 11 amino acids, SEQ ID NO: 35 having a C-terminal truncation of 12 amino acids, SEQ ID NO: 36 having a C-terminal truncation of 13 amino acids, SEQ ID NO: 37 having a C-terminal truncation of 14 amino acids, SEQ ID NO: 38 having a C-terminal truncation of 15 amino acids, and SEQ ID NO: 39 having a C-terminal truncation of 16 amino acids. As the C-terminus of the CTT is the R-domain, the truncation concerns the R-domain of the CTT. In the second embodiment, the glycoproteins have been found suitable for pseudotyping both alpharetroviral vector particles and lentiviral vector particles, and respectively the invention provides alpharetroviral vector particles as well as lentiviral vector particles having the BaEV-derived glycoprotein.

In a third embodiment, the invention provides a glycoprotein originating from MPMV having SEQ ID NO: 43, which can be encoded by the wild-type nucleic acid sequence of SEQ ID NO: 44, which at its 5' end contains additional 6 nucleotides in order to form a Kozak sequence with the downstream nucleotides, and preferably be encoded by a codon-optimized nucleic acid sequence according to SEQ ID NO: 45, which at its 5' end contains an additional 6 nucleotides that form a Kozak sequence with the downstream nucleotides.

In a further embodiment, the invention provides glycoproteins which are hybrids of domains originating from different retroviruses, namely a glycoprotein consisting of the ectodomain and transmembrane domain originating from MPMV and the CTT originating from MLV-A, the glycoprotein having SEQ ID NO: 46, which can be encoded by the wild-type nucleic acid sequence of SEQ ID NO: 47, which at its 5' end contains additional 6 nucleotides to form a Kozak sequence with the downstream nucleotides, and preferably be encoded by a codon-optimized nucleic acid sequence according to SEQ ID NO: 48, which at its 5' end contains additional 6 nucleotides to form a Kozak sequence with the downstream nucleotides.

Another hybrid glycoprotein consists of the ectodomain and transmembrane domain originating from RD114 and the CTT originating from MLV-A, which is truncated to its T-domain, the glycoprotein having SEQ ID NO: 49, and preferably is encoded by a codon-optimized nucleic acid sequence according to SEQ ID NO: 50, which at its 5' end contains additional 6 nucleotides forming part of a Kozak sequence with the downstream nucleotides.

Another hybrid glycoprotein consists of the ectodomain and transmembrane domain originating from RD114 and the CTT originating from MPMV, the glycoprotein having SEQ ID NO: 51, which preferably is encoded by a codon-optimized nucleic acid sequence according to SEQ ID NO: 52, which at its 5' end contains additional 6 nucleotides forming part of a Kozak sequence.

Generally preferred, the nucleic acid sequence encoding the glycoprotein of the invention is directly preceded in 5' by GCCACC in order to form a Kozak sequence with the downstream nucleotides, as it has been found that this enhances transgene expression by improving RNA translatability.

The invention is in the following described by way of examples with reference to the figures that show in
- Fig. 1A a schematic depiction of a retrovirus,
- Fig. 1B a schematic enlarged depiction of a retroviral membrane-bound envelope glycoprotein,
- Fig. 1C a schematic depiction of the arrangement of domains of a retroviral envelope protein,
- Fig. 2 a graphical representation of alpharetroviral vector particle titers obtained in HEK 293T packaging cells,
- Fig. 3 a graphical representation of alpharetroviral vector particle titers obtained in HEK 293T packaging cells,
- Fig. 4 a graphical representation of lentiviral vector particle titers obtained in HEK 293T packaging cells,
- Fig. 5 a graphical representation of alpharetroviral vector particle titers obtained in HEK 293T packaging cells,
- Fig. 6 a graphical representation of alpharetroviral vector particle titers obtained in HEK 293T packaging cells before or after concentration of culture supernatant,
- Fig. 7 a graphical representation of alpharetroviral vector particle titers obtained in HEK 293T packaging cells, and in
- Fig. 8 a graphical representation of lentiviral vector particle titers obtained in HEK 293T packaging cells.

Herein, titers of viral vector particles are determined as titers causing transduction. Of note, transgene (EGFP) expression implies that the vector entry was efficient and that the vector is stably integrated and functionally active.

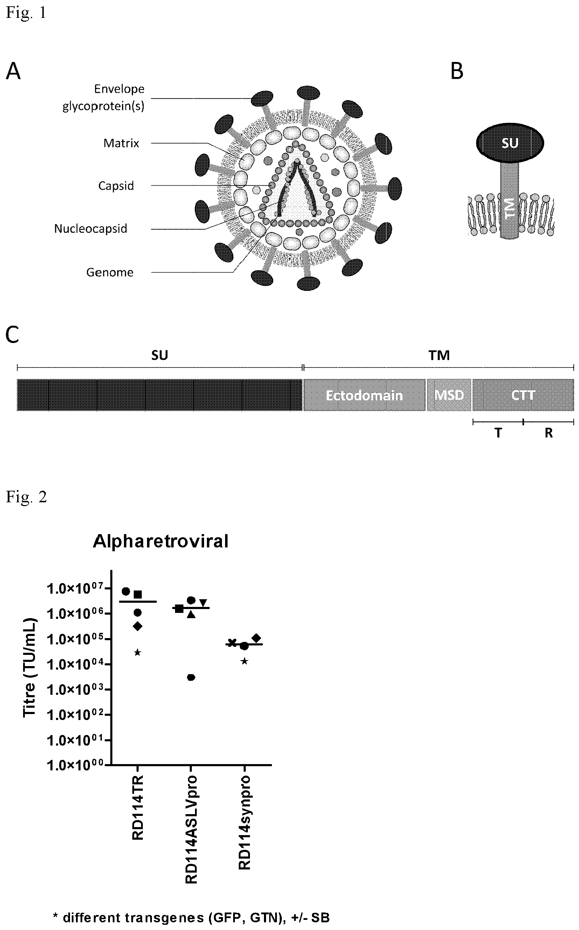

Fig. 1A schematically shows a retroviral vector particle which in its outer membrane envelope contains retroviral glycoproteins (envelope glycoproteins). On the inner side of the membrane envelope, matrix proteins (Matrix) are arranged, which surround the capsid (Capsid) containing the nucleocapsid (Nucleocapsid) including the retroviral genome (Genome).

As shown in Fig. 1B, the retroviral glycoproteins are composed of a surface unit (SU) and a transmembrane (TM) part, which in a packaging cell are initially expressed as a polyprotein and cleaved upon translation within the endoplasmic reticulum. The TM part consists of an ectodomain, harbouring a fusogenic region mediating the fusion with target cells, a membrane-spanning domain (MSD) anchoring the protein within the viral particle envelope, and a C-terminal tail (CTT) (Fig. 1C).

Generally, in the examples, the same vector construct was used in the production of alpharetroviral vector particles or of lentiviral vector particles, with the transgene as indicated in the examples.

Production of the retroviral vector particles was in HEK 293T-packaging cells by the steps of calcium-phosphate-assisted transfection of the cells with plasmids encoding for all required retroviral particle components. The cells are seeded on the day prior to transfection. For transfection, the following plasmids are combined: (I) a transfer vector plasmid encoding for the vector genome, (II) a packaging plasmid encoding for Gag-Pol, and (III) a plasmid encoding for an envelope glycoprotein. In case of the production of lentiviral particles, another plasmid (IV) encoding for Rev is included. Transfection is further assisted by 25 µM chloroquine and performed in the presence of 10-20mM HEPES buffer in standard culture medium. The cells are rinsed 6-12h post-transfection, replacing the medium by standard culture medium supplemented with 10-20mM HEPES. Viral particles are produced by the cells and harvested twice at 32-40h and 44-52h post-transfection by taking off the supernatant and filtering it through a 0.22µm pore size filter. Supernatants are then either directly frozen at -80°C until further usage, or optionally concentrated via ultracentrifugation prior to freezing.

The titer of functional viral vector particles was tested by transducing HT1080 cells by the steps of replacing the medium on the cells by standard culture medium supplemented with 4µg/mL protamine sulphate, followed by addition of the viral particles. Optionally, the viral particles were spin-inoculated by centrifugation for 1h at 711 x g and 32-37 °C. At 6-12h post-transduction, the supernatant was taken off and replaced by standard culture medium. Titers of viral particles were calculated based on the number of seeded cells, the applied volume of viral particle preparation, and the percentage of transgene-positive cells as determined by flow cytometry at 5-7 days post-transduction. Accordingly, the titers given here for viral particles represent fully functional viral vector particles that transduce cells.

The titers obtained in HEK 293T-packaging cells for the chimeric glycoprotein according to EP 1 499 736 B1, referred to as RD114/TR (RD114TR), were used in parallel as a comparison. As a further comparison, a C-terminally truncated variant of RD114/TR, truncated for its 9 C-terminal amino acids (RD114TR-9), was used.

For generation of alpharetroviral and lentiviral vector particles, the SIN vector was cotransfected together with helper plasmids for structural proteins and replication enzymes (Gag/Pol) and envelope glycoproteins (Env). Additionally, for lentiviral particle production a helper plasmid encoding Rev is added. An exemplary packaging plasmid for lentiviral Gag/Pol is SEQ ID NO: 59 (pcDNA3.g/p.4xCTE (11035bp)), an exemplary packaging plasmid for Rev is SEQ ID NO: 60 (pRSV_Rev (4180bp)), the encoded Rev protein is given as SEQ ID NO: 61. An exemplary lentiviral vector transfer plasmid is given as SEQ ID NO: 62. The Examples provide a representative production process by way of exemplary glycoproteins and viral vector particles.

### Example 1: Retroviral envelope glycoproteins based on RD114 having a C-terminally truncated CTT

For producing alpharetroviral vector particles, a plasmid encoding for the viral vector with viral elements (e.g. LTRs, packaging signal) and a transgene expression cassette and a separate expression plasmid for the glycoprotein and an expression plasmid for Gag/Pol was used. As an example for an expression plasmid encoding one of the glycoproteins (Env plasmid), the nucleic acid sequence of SEQ ID NO: 55 or of SEQ ID NO: 57 was used, containing the coding sequence for MPMVco as the transgene. The coding sequence for the retroviral envelope glycoprotein of each of these expression plasmids could be replaced by a coding sequence for the other glycoproteins. These expression plasmids could be used for expressing the glycoprotein both for producing alpharetroviral vector particles and lentiviral vector particles as well as for gammaretroviral vector particles. This production process was employed for producing the other viral vector particles of the invention.

In the process for producing viral particles, HEK293T cells were kept in complete culture medium at 37°C in the presence of 5% CO₂, cells were passaged every 2nd - 3rd day, cells could be rinsed with 1xPBS. Cells were detached by treatment with 1x trypsin/EDTA and incubation for 5 minutes at 37°C, and the reaction was stopped by adding standard culture medium. The culture was split at the respective ratio (usually 1:8-1:12). For viral vector production, HEK293T cells were seeded at day 0 on tissue-culture-grade plastic dishes in standard culture medium. On day 1, transient transfection of the packaging components into HEK293T cells was performed. For this, all packaging components were combined in sterile water.
For lentiviral vector particles, the Gag/Pol packaging construct, the Env plasmid, the Rev plasmid and the vector plasmid were combined.
For alpharetroviral vector particles, the Gag/Pol packaging construct, the Env plasmid and the vector plasmid were combined.

To the packaging components 10% (v/v) [250mM] calcium chloride was added and mixed well, then the DNA/CaCl₂ mixture was added dropwise to an equal volume of 2xHeBS while generating air bubbles with an electronic pipette filler during addition of the DNA mix and for another 30 seconds. The mixture was incubated for 20 minutes at room temperature. Meanwhile, the medium on the cultured cells was replaced by fresh complete culture medium supplemented with 1-2% (v/v) [10-20mM] of HEPES and 0.1% (v/v) [25µM] chloroquine. Upon the 20 minutes incubation, the transfection mix was dropwise added to the cells, the cells were cultured at 37°C and 5% CO₂, and 6-16 h post-transfection, the medium was replaced by fresh complete culture medium supplemented with 10-20mM HEPES.

The basic culture medium was Dulbecco's Modified Eagle's medium (DMEM), with 10% v/v heat inactivated (30 min at 56°C) fetal bovine serum, 10000 U penicillin, 10 mg/mL streptomycin, 1% v/v 100mM sodium pyruvate. For cell detachment, 10x trypsin/EDTA (0.5% trypsin/ 0.2% EDTA) was diluted 1:10 in 1x phosphate buffered saline (PBS).

On day 3: Harvest I: 30-36 hours post-transfection, the medium containing produced viral particles was harvested and passed through a 0.22µM pore size filter, and fresh complete culture medium supplemented with 10-20mM HEPES was added to the transfected cells for a second harvest.

On day 4: Harvest II: 48-54 hours post-transfection, the second harvest was accomplished as per the procedure of the harvest I. The two harvests (I and II) from each plate were pooled. The viral vector preparation was either immediately frozen at -80°C without further concentration or concentrated using ultracentrifugation.

For ultracentrifugation, the viral vector preparation was loaded into SW28 polyallomer tubes. For concentration, the viral vector particles were spun overnight at 10000rpm (rotor SW32Ti) at 4°C. The supernatant was decanted off and the pellet containing the viral particles resuspended in reconstitution buffer consisting of PBS supplemented with 1-2% HEPES or in culture medium. The volume used for resuspension was usually chosen to concentrate the vector preparation 50- to 300- fold. Aliquots were prepared and stored at -80°C until further usage.

The following C-terminal truncations of the CTT of the RD114 glycoprotein were expressed:

| C-terminal truncation | ectodomain | transmembrane domain | CTT |
|---|---|---|---|
| no truncation (RD114wt) | SEQ ID NO: 1 | SEQ ID NO: 2 | SRLMAFINDRLNVVHAM VLAQQYQALKAEEEAQD (SEQ ID NO: 3) |
| C-terminal truncation by 1 amino acid | SEQ ID NO: 1 | SEQ ID NO: 2 | SRLMAFINDRLNVVHAM VLAQQYQALKAEEEAQ (SEQ ID NO: 4) |
| C-terminal truncation by 2 amino acids | SEQ ID NO: 1 | SEQ ID NO: 2 | SRLMAFINDRLNVVHAM VLAQQYQALKAEEEA (SEQ ID NO: 5) |
| C-terminal truncation by 3 amino acids | SEQ ID NO: 1 | SEQ ID NO: 2 | SRLMAFINDRLNVVHAM VLAQQYQALKAEEE (SEQ ID NO: 6) |
| C-terminal truncation by 4 amino acids | SEQ ID NO: 1 | SEQ ID NO: 2 | SRLMAFINDRLNVVHAM VLAQQYQALKAEE (SEQ ID NO: 7) |
| C-terminal truncation by 5 amino acids | SEQ ID NO: 1 | SEQ ID NO: 2 | SRLMAFINDRLNVVHAM VLAQQYQALKAE (SEQ ID NO: 8) |
| C-terminal truncation by 6 amino acids | SEQ ID NO: 1 | SEQ ID NO: 2 | SRLMAFINDRLNVVHAM VLAQQYQALKA (SEQ ID NO: 9) |
| C-terminal truncation by 7 amino acids | SEQ ID NO: 1 | SEQ ID NO: 2 | SRLMAFINDRLNVVHAM VLAQQYQALK (SEQ ID NO: 10) |
| C-terminal truncation by 8 amino acids | SEQ ID NO: 1 | SEQ ID NO: 2 | SRLMAFINDRLNVVHAM VLAQQYQAL (SEQ ID NO: 11) |
| C-terminal truncation by 9 amino acids | SEQ ID NO: 1 | SEQ ID NO: 2 | SRLMAFINDRLNVVHAM VLAQQYQA (SEQ ID NO: 12) |
| C-terminal truncation by 10 amino acids | SEQ ID NO: 1 | SEQ ID NO: 2 | SRLMAFINDRLNVVHAM VLAQQYQ (SEQ ID NO: 13) |
| C-terminal truncation by 11 amino acids | SEQ ID NO: 1 | SEQ ID NO: 2 | SRLMAFINDRLNVVHAM VLAQQY (SEQ ID NO: 14) |
| C-terminal truncation by 12 amino acids | SEQ ID NO: 1 | SEQ ID NO: 2 | SRLMAFINDRLNVVHAM VLAQQ (SEQ ID NO: 15) |
| C-terminal truncation by 13 amino acids | SEQ ID NO: 1 | SEQ ID NO: 2 | SRLMAFINDRLNVVHAM VLAQ (SEQ ID NO: 16) |
| C-terminal truncation by 14 amino acids | SEQ ID NO: 1 | SEQ ID NO: 2 | SRLMAFINDRLNVVHAM VLA (SEQ ID NO: 17) |
| C-terminal truncation by 15 amino acids | SEQ ID NO: 1 | SEQ ID NO: 2 | SRLMAFINDRLNVVHAM VL (SEQ ID NO: 18) |
| C-terminal truncation by 16 amino acids | SEQ ID NO: 1 | SEQ ID NO: 2 | SRLMAFINDRLNVVHAM V (SEQ ID NO: 19) |
| C-terminal truncation by 17 amino acids | SEQ ID NO: 1 | SEQ ID NO: 2 | SRLMAFINDRLNVVHAM (SEQ ID NO: 20) |

As further embodiments, the coding sequence for the retroviral envelope glycoprotein encoded one combination of an ectodomain of SEQ ID NO: 1, a transmembrane domain of SEQ ID NO: 2, and a CTT of one of SEQ ID NO: 41 and SEQ ID NO: 42. The CTT of SEQ ID NO: 41 contains a protease site derived from ASLV, the CTT of SEQ ID NO: 42 contains an artificial protease site.

The resulting titers are shown in Fig. 2 for the glycoproteins having the CTT of SEQ ID NO: 41 or of SEQ ID NO: 42. The symbols each represent results of parallel experiments, each expressing the same transgene EGFP (●, ■, ▲, ◆, ▼, *). This result shows that the glycoprotein having the CTT of SEQ ID NO: 41 yields titers that are comparable to the titers obtained for the reference RD114/TR(RD114TR), and yields higher titers than the glycoprotein having the CTT of SEQ ID NO: 42.

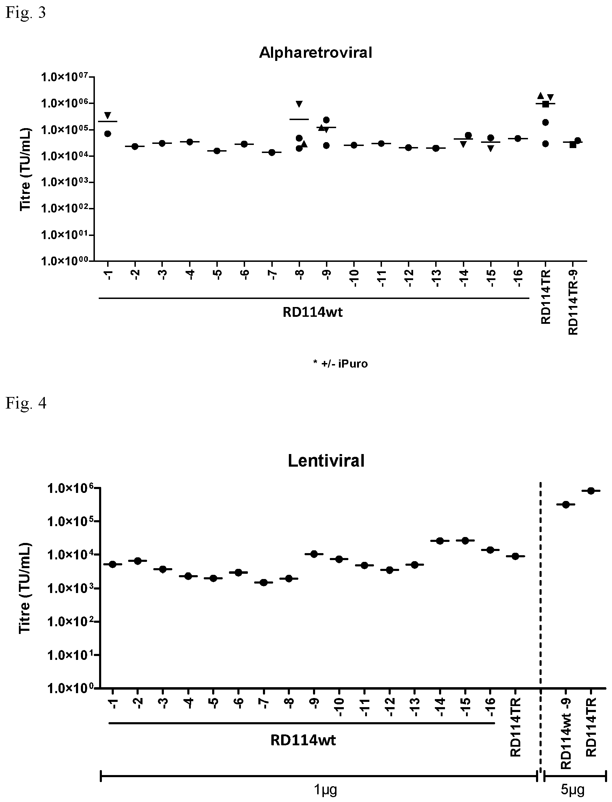

In Fig. 3 and Fig. 4, the truncations of the CTT are indicated as -1 (SEQ ID NO: 4) to -16 (SEQ ID NO: 19) of the wild-type RD114 (RD114wt).

For alpharetroviral vector particles, the resulting titers using the glycoproteins having the truncated CTT of one of SEQ ID NO: 4 to SEQ ID NO: 19 are given in Fig. 3 for 1 µg of plasmid DNA encoding for the respective glycoprotein transfected into packaging cells, showing that for alpharetroviral vector particles, the CTT with a C-terminal truncation of 1 amino acid (CTT of SEQ ID NO: 4), of 8 amino acids (CTT of SEQ ID NO: 11) or of 9 amino acids (CTT of SEQ ID NO: 12) yields higher titers.

For lentiviral vector particles, the resulting titers for pseudotypes using the glycoproteins having the truncated CTT of one of SEQ ID NO: 4 to SEQ ID NO: 19 are given in Fig. 4, showing that for lentiviral vector particles, the CTT with a C-terminal truncation of 9 amino acids (CTT of SEQ ID NO: 12), of 14 amino acids (CTT of SEQ ID NO: 17) or of 15 amino acids (CTT of SEQ ID NO: 18) yields higher titers, also higher than that obtained for the comparative RD 114/TR. It is noted that in Fig. 4, 1 µg plasmid DNA encoding for the respective glycoprotein was used to transfect the packaging cells, and alternatively for the glycoprotein having the CTT of SEQ ID NO: 12 (RD114wt-9) and for comparative RD114/TR, 5 µg plasmid DNA encoding for the respective glycoprotein was used.

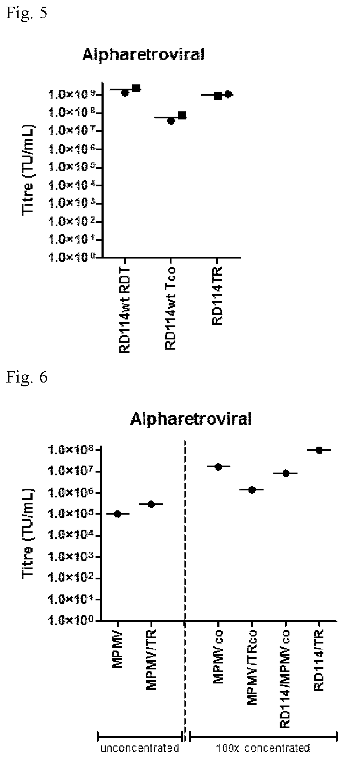

For alpharetroviral vector particles, the resulting titers using the glycoprotein having the truncated CTT of SEQ ID NO: 20 are given in Fig. 5, showing that for alpharetroviral vector particles, the CTT with a C-terminal truncation of 17 amino acids (CTT of SEQ ID NO: 20) yields titers comparable to the reference RD114/TR.

The transgene in each case was EGFP in separate experiments. The individual symbols represent productions tested within the same transduction experiments (●, ■, ▲, ◆, ▼, *).

### Example 2: Hybrid retroviral envelope glycoproteins

The hybrid retroviral envelope glycoproteins of SEQ ID NO: 43, SEQ ID NO: 46, SEQ ID NO: 49 or SEQ ID NO: 51 were encoded on an expression plasmid as described in Example 1. The titers obtained from packaging cells are shown in Fig. 5 and Fig. 6.

The results show that the glycoprotein of SEQ ID NO: 43 (MPMV) and of SEQ ID NO: 46 (MPMV/TR) prior to any concentrating step reached 10⁵ to 5 x 10⁵ transducing units/mL. For the production from a codon-optimized coding sequence a 100-fold concentrating step by ultracentrifugation was employed. The titers show that it is optional to produce the glycoprotein of SEQ ID NO: 43 (MPMVco) from a codon-optimized (co) coding sequence of SEQ ID NO: 45, to produce the glycoprotein of SEQ ID NO: 46 (MPMV/TRco) from a codon-optimized (co) coding sequence of SEQ ID NO: 48, to produce the glycoprotein of SEQ ID NO: 49 (RD114 Tco) from a codon-optimized (co) coding sequence of SEQ ID NO: 50, and to produce the glycoprotein of SEQ ID NO: 51 (RD114/MPMVco) from a codon-optimized (co) coding sequence of SEQ ID NO: 52.

### Example 3: Retroviral envelope glycoproteins based on BaEV having a C-terminally truncated CTT

For producing alpharetroviral vector particles or lentiviral vector particles containing the glycoproteins indicated below a plasmid corresponding to SEQ ID NO: 55 or SEQ ID NO: 57 with the glycoprotein encoding sequence exchanged for a coding sequence encoding these amino acid sequences was used.

The following C-terminal truncations of the CTT of the BaEV glycoprotein were expressed:

| C-terminal truncation | ectodomain | transmembrane domain | CTT |
|---|---|---|---|
| no truncation (BaEVwt) | SEQ ID NO: 21 | SEQ ID NO: 22 | NRLTAFINDKLNIIHAMVLTQQYQ VLRTDEEAQD (SEQ ID NO: 23) |
| C-terminal truncation by 1 amino acid | SEQ ID NO: 21 | SEQ ID NO: 22 | NRLTAFINDKLNIIHAMVLTQQYQ VLRTDEEAQ (SEQ ID NO: 24) |
| C-terminal truncation by 2 amino acids | SEQ ID NO: 21 | SEQ ID NO: 22 | NRLTAFINDKLNIIHAMVLTQQYQ VLRTDEEA (SEQ ID NO: 25) |
| C-terminal truncation by 3 amino acids | SEQ ID NO: 21 | SEQ ID NO: 22 | NRLTAFINDKLNIIHAMVLTQQYQ VLRTDEE (SEQ ID NO: 26) |
| C-terminal truncation by 4 amino acids | SEQ ID NO: 21 | SEQ ID NO: 22 | NRLTAFINDKLNIIHAMVLTQQYQ VLRTDE (SEQ ID NO: 27) |
| C-terminal truncation by 5 amino acids | SEQ ID NO: 21 | SEQ ID NO: 22 | NRLTAFINDKLNIIHAMVLTQQYQ VLRTD (SEQ ID NO: 28) |
| C-terminal truncation by 6 amino acids | SEQ ID NO: 21 | SEQ ID NO: 22 | NRLTAFINDKLNIIHAMVLTQQYQ VLRT (SEQ ID NO: 29) |
| C-terminal truncation by 7 amino acids | SEQ ID NO: 21 | SEQ ID NO: 22 | NRLTAFINDKLNIIHAMVLTQQYQ VLR (SEQ ID NO: 30) |
| C-terminal truncation by 8 amino acids | SEQ ID NO: 21 | SEQ ID NO: 22 | NRLTAFINDKLNIIHAMVLTQQYQ VL (SEQ ID NO: 31) |
| C-terminal truncation by 9 amino acids | SEQ ID NO: 21 | SEQ ID NO: 22 | NRLTAFINDKLNIIHAMVLTQQYQ V (SEQ ID NO: 32) |
| C-terminal truncation by 10 amino acids | SEQ ID NO: 21 | SEQ ID NO: 22 | NRLTAFINDKLNIIHAMVLTQQYQ (SEQ ID NO: 33) |
| C-terminal truncation by 11 amino acids | SEQ ID NO: 21 | SEQ ID NO: 22 | NRLTAFINDKLNIIHAMVLTQQY (SEQ ID NO: 34) |
| C-terminal truncation by 12 amino acids | SEQ ID NO: 21 | SEQ ID NO: 22 | NRL TAFINDKLNIIHAMVL TQQ (SEQ ID NO: 35) |
| C-terminal truncation by 13 amino acids | SEQ ID NO: 21 | SEQ ID NO: 22 | NRLTAFINDKLNIIHAMVLTQ (SEQ ID NO: 36) |
| C-terminal truncation by 14 amino acids | SEQ ID NO: 21 | SEQ ID NO: 22 | NRLTAFINDKLNIIHAMVLT (SEQ ID NO: 37) |
| C-terminal truncation by 15 amino acids | SEQ ID NO: 21 | SEQ ID NO: 22 | NRLTAFINDKLNIIHAMVL (SEQ ID NO: 38) |
| C-terminal truncation by 16 amino acids | SEQ ID NO: 21 | SEQ ID NO: 22 | NRLTAFINDKLNIIHAMV (SEQ ID NO: 39) |
| C-terminal truncation by 17 amino acids | SEQ ID NO: 21 | SEQ ID NO: 22 | NRLTAFINDKLNIIHAM (SEQ ID NO: 40) |

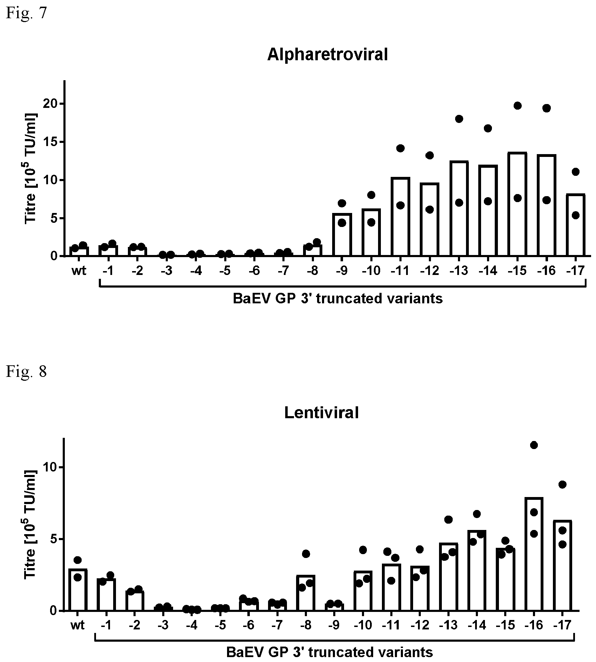

For alpharetroviral vector particles, the resulting titers for the glycoproteins having the truncated CTT of one of SEQ ID NO: 23 to SEQ ID NO: 40 are given in Fig. 7 for 1 µg plasmid DNA encoding for the respective glycoprotein transfected during production, showing that in alpharetroviral vector particles, the CTT with a C-terminal truncation of 11 amino acids (CTT of SEQ ID NO: 34) to a C-terminal truncation of 16 amino acids (CTT of SEQ ID NO: 39) yields higher titers.

For lentiviral vector particles, the resulting titers when using the glycoproteins having the truncated CTT of one of SEQ ID NO: 23 to SEQ ID NO: 40 are given in Fig. 8 for 1 µg plasmid DNA encoding for the respective glycoprotein transfected during production,

## Claims

1. Retroviral vector particle, which is an alpharetroviral vector particle or a lentiviral vector particle or a gammaretroviral vector particle, the vector particle comprising in its envelope: (i) a retroviral envelope glycoprotein comprising an ectodomain having the amino acid sequence of SEQ ID NO: 1, a transmembrane domain having the amino acid sequence of SEQ ID NO: 2, and one C-terminal tail (CTT) having the amino acid sequence selected from SEQ ID NO: 4, SEQ ID NO: 11, SEQ ID NO: 12, SEQ ID NO: 17, SEQ ID NO: 18, SEQ ID NO: 20, SEQ ID NO: 41, and SEQ ID NO: 42, or (ii) a retroviral envelope glycoprotein comprising an ectodomain having the amino acid sequence of SEQ ID NO: 21, a transmembrane domain having the amino acid sequence of SEQ ID NO: 22, and one CTT having the amino acid sequence selected from SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40, or (iii) a retroviral envelope glycoprotein having the amino acid sequence of SEQ ID NO: 43, of SEQ ID NO: 46, of SEQ ID NO: 49, or of SEQ ID NO: 51.

2. Retroviral vector particle according to claim 1, **characterized in that** the envelope glycoprotein binds to SLC1A5 receptor for cell entry.

3. Retroviral vector particle according to one of the preceding claims for use in the genetic treatment of human cells.

4. Retroviral vector particle according to one of the preceding claims for use in the genetic treatment of human cells, wherein the use is treatment of a genetic defect.

5. Retroviral vector particle according to one of the preceding claims, **characterized in that** the vector particle is an alpharetroviral particle and the retroviral envelope glycoprotein consists of one amino acid sequence comprising the ectodomain of SEQ ID NO: 1, the transmembrane domain of SEQ ID NO: 2 and one CTT of the group of SEQ ID NO: 4, SEQ ID NO: 11, and SEQ ID NO: 12.

6. Retroviral vector particle to one of the preceding claims, **characterized in that** the vector particle is a lentiviral particle and the retroviral envelope glycoprotein consists of one amino acid sequence comprising the ectodomain of SEQ ID NO: 1, the transmembrane domain of SEQ ID NO: 2 and one CTT of the group of SEQ ID NO: 17, SEQ ID NO: 18, and SEQ ID NO: 20.

7. Retroviral vector particle according to one of the preceding claims, **characterized in that** the vector particle is an alpharetroviral particle and the retroviral envelope glycoprotein consists of one amino acid sequence comprising the ectodomain of SEQ ID NO: 21, the transmembrane domain of SEQ ID NO: 22 and one CTT of the group of SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40.

8. Retroviral vector particle to one of the preceding claims, **characterized in that** the vector particle is a lentiviral particle and the retroviral envelope glycoprotein consists of one amino acid sequence comprising the ectodomain of SEQ ID NO: 21, the transmembrane domain of SEQ ID NO: 22 and one CTT of the group of SEQ ID NO: 31, SEQ ID NO: 33, SEQ ID NO: 34, SEQ ID NO: 35, SEQ ID NO: 36, SEQ ID NO: 37, SEQ ID NO: 38, SEQ ID NO: 39, and SEQ ID NO: 40.

9. Retroviral vector particle to one of the preceding claims, **characterized in that** the vector particle is a viral particle based on ASLV.

10. Process for transducing human cells by introducing a retroviral vector particle encoding a transgene, **characterized in that** the retroviral vector particle is one according to one of the preceding claims.

11. Process for producing a retroviral vector particle according to claim 10 by expressing the retroviral vector particle from plasmids contained in a packaging cell.
